# EUROPÄISCHE PATENTSCHRIFT

(11) **EP 2 134 195 B1**
(45) Veröffentlichungstag und Bekanntmachung des Hinweises auf die Patenterteilung: **02.11.2011**
(21) Anmeldenummer: 08717407.4
(22) Anmeldetag: 05.03.2008
(51) Int. Cl.: A23L 1/236, A23L 1/22

(54) **VERFAHREN ZUR GESCHMACKSMODULATION VON STOFFLICHEN ZUSAMMENSETZUNGEN, DIE MINDESTENS EINEN HIGH INTENSITY SWEETENER (HIS) ENTHALTEN**
METHOD FOR MODULATING THE TASTE OF MATERIAL COMPOSITIONS CONTAINING AT LEAST ONE HIGH INTENSITY SWEETENER (HIS)
PROCÉDÉ DE MODULATION DU GOÛT DE COMPOSITIONS DE MATIÈRES QUI CONTIENNENT AU MOINS UN ÉDULCORANT DE HAUTE INTENSITÉ

(43) Veröffentlichungstag der Anmeldung: 23.12.2009
(73) Patentinhaber: B.R.A.I.N. Biotechnology Research And Information Network AG, 64673 Zwingenberg (DE)
(72) Erfinder: MATUSCHEK, Markus, 69469 Weinheim (DE); ZINKE , Holger, 64673 Zwingenberg (DE); KROHN, Michael, 64653 Lorsch, (DE); KLEBER, Alice, 64625 Bensheim, (DE); JAGER, Martin, B., 67677 Enkenbach-Alsenbom, (DE)
(74) Vertreter: Vossius & Partner
(86) Internationale Anmeldenummer: PCT/EP2008/052657
(87) Internationale Veröffentlichungsnummer: WO 2009/109227

(56) Entgegenhaltungen:
- EP-A- 0 750 849
- WO-A-2007/061795
- WO-A-2007/135362
- JP-A- 2007 254 584
- US-A- 4 208 444
- US-A1- 2004 191 298

## Beschreibung

### Gebiet der Erfindung

Die vorliegende Erfindung betrifft ein neues Verfahren zur Geschmacksmodulation, insbesondere zur Reduktion von bitterem Geschmack und Nachgeschmack, von stofflichen Zusammensetzungen, die mindestens einen High Intensity Sweetener (HIS) enthalten.

### Stand der Technik

Stoffliche Zusammensetzungen wie Lebensmittel, Getränke, Genussmittel, Süßungsmittel, Tiernahrungsmittel, Kosmetika und Pharmazeutika enthalten häufig Geschmacksstoffe, die grundsätzlich unerwünscht oder in der vorhandenen Intensität zu dominant oder zu niedrig sind. Im Bereich der Süßungsmittel treten häufig neben den süßen Geschmackseindrücken weitere Geschmackseindrücke wie z.B. ein metallischer, chemischer, bitterer oder synthetischer Geschmack oder Nachgeschmack auf, die den Gesamtgeschmackseindruck der zu süßenden Zusammensetzung nachteilig beeinflussen. Im Rahmen der vorliegenden Erfindung wird unter Geschmack der unmittelbare Geschmackseindruck verstanden, der entsteht, während sich die Zusammensetzung im Mund befindet. Unter Nachgeschmack wird die Geschmackswahrnehmung nach dem Abschlucken, insbesondere nach einer Wartezeit von etwa 30 Sekunden, verstanden.

Beispielsweise sind Koffein in Tee oder Kaffee sowie Hopfenextrakte in Bier natürliche Bitterstoffe, welche jedoch in zu hoher Konzentration einen negativen Geschmackseindruck hervorrufen. In speziellen Bittergetränken wie beispielsweise Tonic Water oder Bitter Lemon ist ein charakteristischer Bittergeschmack, hervorgerufen durch den Zusatzstoff Chinin, in besonderem Maße erwünscht. Andererseits ist insbesondere der bittere Geschmack oder Nachgeschmack vieler künstlicher Süßstoffe wie beispielsweise Acesulfam K (ACK) und Saccharin ein unerwünschter Nebengeschmack des Süßstoffes in anderen Limonaden. Auch Fruchtsäfte, insbesondere Orangensaft, leiden unter der Beeinträchtigung des Geschmackes durch z.B. Flavonoidglykoside, welche einen bitteren Geschmack haben. Auch viele pharmazeutische Wirkstoffe, insbesondere Ibuprofen haben einen stark bitteren Geschmack, welcher zur Reduktion der Akzeptanz bei der Einnahme des Wirkstoffes führt.

Zur Reduktion des natürlichen Bittergeschmacks beispielsweise von Tee, Kaffee oder Orangensaft werden diese Lebensmittel, Getränke, Genussmittel entweder enzymatisch behandelt, um die bitter schmeckenden Substanzen zu zerstören, oder die bittere Substanz wird wie im Falle des Koffeins im Tee bzw. Kaffee durch Entkoffeinierung entfernt.

Eine andere Möglichkeit besteht darin, den bitter schmeckenden Wirkstoff durch Formulierungstechniken wie z.B. in Tabletten einzuschließen. Der bitter schmeckende Wirkstoff wird dann nicht im Mund freigesetzt, sondern erst im Magen-Darm-Trakt.

Eine weitere Möglichkeit der Modifikation des Geschmackseindrucks stellt die Zugabe von Geschmacksmodulatoren oder Aromen zu den gewünschten Lebensmitteln, Getränken, Genussmitteln, Tiernahrungsmitteln, Süßungsmitteln, Kosmetika und Pharmazeutika dar.

Beispielsweise stellt die internationale Patentanmeldung WO 2007/061735 allgemein natürliche Süßmittelzusammensetzungen , mit hoher Süßkraft, verbessertem Zeit- und/oder Geschmacksprofil bereit. Genauer gesagt umfassen die natürlichen Süßmittelzusammensetzungen Rebaudiosid-A und ein Polyol.

Es ist daher wünschenswert, Stoffe zu finden, die unangenehme Geschmackseindrücke unterdrücken oder reduzieren sowie gewünschte Geschmackseindrücke gezielt verstärken oder zumindest nicht negativ beeinflussen.

Insbesondere im Bereich der Pharmawirkstoffe sind eine ganze Reihe von, insbesondere bittermodifizierenden, Substanzen bekannt. So wird beispielsweise der bittere Geschmack von Ibuprofen durch Polylysine und Polyarginine (vgl. die internationale Patentanmeldung WO 2003/086293), durch Meglumin-Salz (vgl. das amerikanische Patent US 5,028,625), durch Natriumchlorid oder Natrium-Sacharin (vgl. die internationale Patentanmeldung WO 2003/047550) oder durch Hydroxypropyl-beta-cyclodextrin oder kaubare Methacrylsäurecopolymerisate (vgl. Modifying Bitterness, Mechanism, Ingredients And Applications, Glenn Roy, 1997) maskiert, um die Einnahme durch die Patienten zu erleichtern. Auch Koffein lässt sich durch eine Vielzahl von Geschmacksmodulatoren in seiner Bitterkeit reduzieren wie beispielsweise durch Glutaminsäure, Dicalcium-disalicylat, Stärke, Lactose, Manitol sowie durch Phosphatidsäure und beta-Lactoglobulin (vgl. Glenn Roy, 1997) und weiterhin durch Hydroxybenzoesäureamide insbesondere Hydroxybenzoesäurevanillylamide (vgl. Ley et al., Journal of Agricultural & Food Chemistry, 2006).

Weitere Stoffe, die zur Reduktion eines Bittergeschmacks im Allgemeinen und insbesondere in Pharmazeutika und Lebensmittel eingesetzt wurden, sind Lecithin, Ascorbat und Citrat (vgl. die japanische Patentanmeldung JP 2001226293), Ester von Mono-oder Diglyceriden wie Glycerinmonostearat und Polycarbonsäuren wie Succinsäure (vgl. die europäische Patentanmeldung EP 0 732 064 A1), Hydroxyflavanone (vgl. die europäische Patentanmeldung EP 1 258 200 A1), 2-Phenyl-4-chromanonderivate (vgl. die deutsche Patentanmeldung DE 101 22 898), Natriumsulfathydrat (vgl. die japanische Patentanmeldung JP 02025428). Aus dem amerikanischen Patent US 5,637,618 ist weiterhin die Verwendung von Benzoesäurederivaten zur Reduktion des Bittergeschmacks in Getränken sowie von Süßstoffen und von Kaliumchlorid bekannt. Der Bittergeschmack von Kaliumchlorid wird auch mit Hilfe von 2,4-Dihydroxybenzoesäure, Carrageenan und Thaumatin gehemmt (vgl. Glenn Roy, 1997; das amerikanische Patent US 5,637,618 sowie die japanischen Patentanmeldungen JP 04262758 und JP 07083684).

Die bekannten Geschmacksmodulatoren befriedigen aber nicht in vollem Umfang, insbesondere wenn sie zur Reduktion des Bittergeschmacks von stofflichen Zusammensetzungen wie z.B. Lebensmittel, Getränke, Genußmittel, Süßungsmittel, Tiernahrungsmittel, Kosmetika und Pharmazeutika, die mindestens einen HIS enthalten, insbesondere von HIS enthaltenden Erfrischungsgetränken, verwendet werden sollen. Ihre die Bitterkeit reduzierende Wirkung ist häufig nicht ausreichend. Wird aus diesem Grunde die Konzentration der bekannten Geschmacksmodulatoren erhöht, um eine ausreichende Wirkung zu erzielen, kann es zu unerwünschten physiologischen oder physikalischen und/oder chemischen Wechselwirkungen mit den übrigen Bestandteilen der jeweiligen Zusammensetzungen und/oder zu einer nachteiligen Beeinflussung, insbesondere zu einer Verschlechterung bis hin zu einer völligen Verfälschung ihres charakteristischen Geschmackseindrucks, kommen.

### Aufgabe der Erfindung

Demgemäß lag der vorliegenden Erfindung die Aufgabe zu Grunde, ein neues Verfahren zur Geschmacksmodulation insbesondere zur Reduktion von bitterem Geschmack und Nachgeschmack, von stofflichen Zusammensetzungen, die mindestens einen High Intensity Sweetener (HIS) enthalten, insbesondere von Lebensmitteln, Getränken, Genussmitteln, Süßungsmitteln, Tiernahrungsmitteln, Kosmetika und Pharmazeutika, die mindestens einen HIS enthalten, zu finden.

Das neue Verfahren zur Geschmacksmodulation soll bewirken, dass durch die eingesetzten Geschmacksmodulatoren keine unerwünschten physiologischen oder physikalischen und/oder chemischen Wechselwirkungen mit den übrigen Bestandteilen der jeweiligen Zusammensetzungen, insbesondere der Lebensmittel, Getränke, Genussmittel, Süßungsmittel, Tiernahrungsmitteln, Kosmetika und Pharmazeutika, hervorgerufen werden und der charakteristische Geschmackseindruck nicht nachteilig beeinflusst, insbesondere nicht verschlechtert oder völlig verfälscht wird.

Insbesondere soll es das neue Verfahren ermöglichen, den bitteren Geschmack und den bitteren Nachgeschmack von stofflichen Zusammensetzungen, die mindestens einen HIS enthalten, signifikant zu reduzieren.

### Erfindungsgemäße Lösung

Demgemäß wurde das neue Verfahren zur Geschmacksmodulation von stofflichen Zusammensetzungen, die mindestens einen High Intensity Sweetener HIS enthalten "wobei es sich bei dem HIS um Saccharin (SAC) handelt" gefunden, bei dem einer stofflichen Zusammensetzung, die mindestens einen HIS enthält, ein Polyoxyethylenfettsäureester zugesetzt wird.

Im Folgenden wird das neue Verfahren zur Geschmacksmodulation von stofflichen Zusammensetzungen als »erfindungsgemäßes Verfahren« bezeichnet.

### Vorteile der Erfindung

Im Hinblick auf den Stand der Technik war es überraschend und für den Fachmann nicht vorhersehbar, dass die Aufgabe, die der vorliegenden Erfindung zu Grunde lag, mithilfe des erfindungsgemäßen Verfahrens gelöst werden konnte.

Überraschenderweise bewirkte das erfindungsgemäße Verfahren, dass durch die eingesetzten Geschmacksmodulatoren keine unerwünschten physikalischen und/oder chemischen Wechselwirkungen mit den übrigen Bestandteilen der jeweiligen stofflichen Zusammensetzungen, die mindestens einen HIS enthielten "wobei es sich bei dem HIS um Saccharin (SAC) handelt" insbesondere der Lebensmittel, Getränke, Genussmittel, Süßungsmittel, Tiernahrungsmitteln, Kosmetika und Pharmazeutika, hervorgerufen wurde und ihr charakteristischer Geschmackseindruck nicht nachteilig beeinflusst, insbesondere nicht verschlechtert oder gar völlig verfälscht wurde.

Insbesondere ermöglichte es das erfindungsgemäße Verfahren, den bitteren Geschmack und den bitteren Nachgeschmack von stofflichen Zusammensetzungen, die mindestens einen HIS enthitelten "wobei es sich bei dem HIS um Saccharin (SAC) handelt", bereits mit sehr geringen Mengen an Geschmacksmodulatoren signifikant zu reduzieren.

Insbesondere beeinflussten diese überraschend geringen Mengen auch den Farbeindruck der stofflichen Zusammensetzungen nicht nachteilig, was insbesondere im Falle von farbigen Erfrischungsgetränken ein ganz besonderer Vorteil war.

Vor allem war es überraschend, dass die Geschmacksmodulation einer gegebenen stofflichen Zusammensetzung, die mindestens einen HIS enthielt "wobei es sich bei dem HIS um Saccharin (SAC) handelt" durch das erfindungsgemäße Verfahren hervorragend reproduzierbar war, was gerade im Hinblick auf die Herstellung von Massenprodukten wie Lebensmittel, Getränke, Genussmittel, Süßungsmittel, Tiernahrungsmittel, Kosmetika und Pharmazeutika ein ganz besonderer Vorteil war.

### Ausführlich Beschreibung der Erfindung

Das erfindungsgemäße Verfahren betrifft die Geschmacksmodulation, insbesondere die Reduktion des bitteren Geschmacks und des bitteren Nachgeschmacks, von stofflichen Zusammensetzungen, die mindestens einen so genannten High Intensity Sweetener (HIS) enthalten "wobei es sich bei dem HIS um Saccharin (SAC) handelt".

Vorzugsweise handelt es sich bei den stofflichen Zusammensetzungen um Lebensmittel, Getränke Genussmittel, Süßungsmittel, Tiernahrungsmittel, Kosmetika und Pharmazeutika. Bevorzugt handelt es sich bei den Getränken um Erfrischungsgetränke, besonders bevorzugt coffeinhaltige Erfrischungsgetränke, insbesondere Cola-Getränke.

Die stofflichen Zusammensetzungen enthalten mindestens einen High Intensity Sweetener HIS als Süßungsmittel oder Süßstoft "wobei es sich bei dem HIS um Saccharin (SAC) handelt". Unter HIS versteht man Verbindungen synthetischer oder natürlicher Herkunft, die keinen oder im Verhältnis zur Süßkraft vernachlässigbaren physiologischen Brennwert besitzen (non-nutritive sweeteners) und eine um ein Vielfaches höhere Süßkraft als Saccharose aufweisen. Die Süßkraft einer Verbindung ist durch die Verdünnung gegeben, bei der sie ebenso süß wie eine Saccharose-Lösung schmeckt (isosüße Lösung; 0,1 M = 4%), d. h. eine 500fach verdünnte Lösung eines Süßstoffs schmeckt isosüß wie eine Saccharose-Lösung, wenn der Süßstoff eine Süßkraft von 500 hat.

Beispiele geeigneter HIS sind aus Römpp Online 2007, »Süßstoffe« bekannt. Erfindungsgemäß ist der Saccharin (SAC)

Vorzugsweise handelt es sich bei der stofflichen Zusammensetzung um eine zuckerarme Zusammensetzung, die weniger als 10 g, vorzugsweise weniger als 1 g, Zucker pro Liter bzw. pro kg Zusammensetzung enthält, insbesondere um eine zuckerfrei Zusammensetzung. Unter Zucker werden vorliegend insbesondere aber nicht aussschliesslich Mono- und Di-Saccharide verstanden.

Vorteilhafterweise handelt es sich bei der stofflichen Zusammensetzung um eine Zusammensetzung mit weniger als 100 kJ, vorzugsweise weniger als 10 kJ, pro Liter oder kg Zusammensetzung.

Vorteilhafterweise handelt es sich bei der stofflichen Zusammensetzung um eine kohlenhydratfreie, insbesondere stärkefreie, Zusammensetzung.

Vorzugsweise handelt es sich bei der stofflichen Zusammensetzung um eine fettarme Zusammensetzung, die weniger als 1 g Fett pro Liter bzw. pro kg Zusammensetzung enthält, insbesondere um eine fettfreie Zusammensetzung.

Bei den zuckerarmen und/oder fettarmen, insbesondere bei den zuckerfreien und/oder fettfreien, Zusammensetzungen handelt es sich vorzugsweise um eine SAC gesüsste Zusammensetzung, insbesondere um ein SAC gesüßtes Getränk.

Vorzugsweise handelt es sich bei dem erfindungsgemäßen Fettsäureester um einen Mono- oder Tri-Fettsäureester, besonders bevorzugt um einen Mono-Fettsäureester.

Bei den Fettsäuren kann es sich sowohl um gesättigte wie mindestens einfach ungesättigte Fettsäuren handeln. Vorzugsweise handelt es sich um gesättigte Fettsäuren.

Bei den Fettsäuren der erfindungsgemäßen Polyoxyethylensorbitanfettsäureestern handelt es sich gemäß einer Ausführungsform um C11- bis C18-Fettsäuren, wobei bevorzugt eine Fettsäure aus der Gruppe bestehend aus Laurinsäure, Ölsäure, Palmitinsäure und Stearinsäure ausgewählt wird. Ganz besonders bevorzugt wird ein Palmitinsäureester, insbesondere ein Polyoxyethylensorbitanmonopalmitin-säureester, verwendet.

Bei den erfindungsgemäßen Fettsäureestern handelt es sich bevorzugt um die in der europäischen Union zugelassenen Lebensmittelzusatzstoffe E432 (Polysorbat 20), E433 (Polysorbat 80), E434 (Polysorbat 40), E435 (Polysorbat 60) oder E436 (Polysorbat 65), ganz besonders bevorzugt um E434.

Gemäß einer Ausführungsform des erfindungsgemäßen Verfahrens wird der mindestens eine Polyoxyethylensorbitanfettsäureester in einer Menge zugesetzt, sodass in den stofflichen Zusammensetzungen letztlich eine Konzentration je Fettsäureester von 1 bis 100 mg, insbesondere 10 bis 15 mg, pro Liter bzw. Kilogramm Zusammensetzung enthalten ist. Besonders bevorzugt enthält die stoffliche Zusammensetzung 13 mg an Fettsäureester, insbesondere an E434, pro Liter bzw. Kilogramm Zusammensetzung, bevorzugt pro Liter Getränk.

Ganz besonders bevorzugt handelt es sich um eine zuckerfreie Zusammensetzung, insbesondere ein Getränk, die SAC und einen Polyoxyethylenfettsäureester, vorzugsweise E434, enthält.

Außer den vorstehend beschriebenen, erfindungsgemäß zu verwendenden Polyoxyethylenfettsäureestem können noch andere übliche und bekannte geschmacksmodulierende Stoffe in wirksamen Mengen eingesetzt werden. Beispiele geeigneter üblicher und bekannter geschmacksmodulierender Stoffe sind die eingangs beschriebenen.

Ein weiterer Aspekt der Erfindung stellt die Verwendung mindestens eines Polyoxyethylenfettsäureesters als Geschmacksmodulator in stofflichen Zusammensetzung, die mindestens einen High Intensity Sweetener (HIS) als Süßungsmittel enthalten, "dadurch gekennzeichnet, dass es sich bei dem HIS um Saccharin (SAC) handelt," dar. Bei den dabei verwendeten Polyoxyethylenfettsäureestern handelt es sich mit Vorteil um die oben im Zusammenhang mit dem erfindungsgemäßen Verfahren beschriebenen Polyoxyethylenfettsäureester und deren besonderen Ausführungsformen. Hinsichtlich der stofflichen Zusammensetzung handelt es sich insbesondere um die oben im Zusammenhang mit dem erfindungsgemäßen Verfahren beschriebenen Ausführungsformen der stofflichen Zusammensetzungen.

Die beschriebenen Ausführungsformen dienen zur Erläuterung und zum besseren Verständnis der Erfindung und sind in keiner Weise einschränkend zu verstehen. Weitere Merkmale der Erfindung ergeben sich aus der nachfolgenden Beschreibung von bevorzugten Ausführungsformen in Verbindung mit den Unteransprüchen. Hierbei können die einzelnen Merkmale der Erfindung bei einer Ausführungsform jeweils einzeln oder zu mehreren verwirklicht sein und stellen in keiner Weise eine Beschränkung der Erfindung auf die beschriebene Ausführungsform dar. Der Wortlaut der Patentansprüche wird hiermit ausdrücklich zum Gegenstand der Beschreibung gemacht.

### Beispiele

### Beispiel 1

Die Verwendung von Polyoxyethylensorbitanfettsäureester zur Geschmacksmodulation von SAC Für Beispiel 1 wurden die folgenden Stoffe verwendet.

HIS:
Saccharin (SAC) von Sigma-Aldrich Chemie GmbH, Taufkirchen, Deutschland;

Polyoxyethylensorbitanfettsäureester:
Tween 40 (E434) von Sigma-Aldrich Chemie GmbH, Taufkirchen, Deutschland;

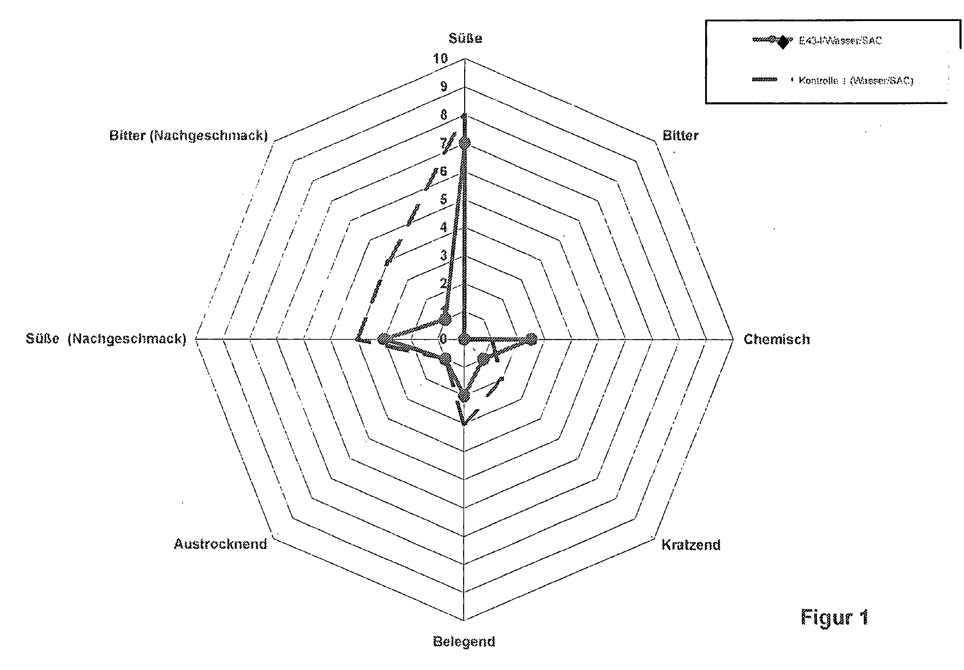

Quantitative sensorische Prüfung - allgemeine Testvorschrift:
Von Probe 1 des Beispiels 1 sowie der Kontrollprobe 1 wurden Konsensprofile in Übereinstimmung mit DIN 10967-2/ISO 11035 erstellt. Dazu wurden 8 trainierte Probanden, die gemäß den DIN/ISO-Vorschriften ausgewählt worden waren, mit den Produkten durch Definition und Trainieren der vorgegebenen Merkmalseigenschaften vertraut gemacht. Anschließend verkosteten die Probanden die Probe 1, um den Geschmack, den Nachgeschmack und das Mundgefühl entsprechend den vorgegebenen Merkmalseigenschaften zu bewerten. Die jeweiligen Konsensprofile wurden vom Prüfungsleiter in der Form von Tabellen und sogenannten Spiderplot-Diagrammen (Figur 1) zusammengefasst. Im Folgenden werden der Übersichtlichkeit halber die Ergebnisse auch als Tabelle wiedergegeben.

Probe 1 und Kontrollprobe 1 - stoffliche Zusammensetzung:
Die Probe 1 und die Kontrollprobe 1 wiesen die nachstehend beschriebenen stofflichen Zusammensetzungen auf. Die jeweiligen Abkürzungen, die in den nachfolgenden Tabellen verwendet werden, werden in Klammern angegeben.

### Kontrollprobe 1:

Wasser + 180 mg/l SAC
(Abkürzung: Wasser/SAC)

### Probe 1 - Beispiel 1:

Wasser + 180 mg/l SAC + 13 mg/l E434
(Abkürzung: Wasser/SAC/E434)

Probe 1 und Kontrollprobe 1 - Versuchsergebnisse:
Die Ergebnisse der quantitativen sensorischen Prüfung der Probe 1 und der Kontrollprobe 1 sind in der Tabelle 1 zusammengestellt.
Bei beiden Tabellen bedeutet der Messwert 0, dass die betreffende sensorische Eigenschaft nicht vorhanden war, während der Messwert 10 bedeutet, dass die betreffende sensorische Eigenschaft stark vorhanden war.

**Tabelle 1: Quantitative sensorische Prüfung der Probe 1 und der Kontrollprobe 1 - Konsensprofile**

| Kontrollprobe/ | Geschmack | | Mundgefühl | | | | Nachgeschmack | |
|---|---|---|---|---|---|---|---|---|
| Probe | S^{a)} | Bt^{b)} | C^{c)} | K^{d)} | Bl^{e)} | A^{f)} | | S(N)^{g)} Bt(N)^{h)} |
| | | | | | | | | |
| Kontrollprobe 1 | | | | | | | | |
| Wasser/SAC | 8 | 0 | 1 | 2 | 3 | 1 | 4 | 4 |
| | | | | | | | | |
| Probe 1 | | | | | | | | |
| Wasser/SAC/ | | | | | | | | |
| E434 | 7 | 0 | 2,5 | | 2 | 1 | 3 | 1 |
| | | | | | | | | |

| | | | | | | | | |
|---|---|---|---|---|---|---|---|---|
| a) S = süß; b) Bt = bitter; c) C = chemisch; d) K = kratzend; e) BI = belegend; f) A = austrocknend; g) S(N) = süßer Nachgeschmack; h) Bt(N) = bitterer Nachgeschmack; | | | | | | | | |

Die Ergebnisse der Tabelle 1 und der Figur 1 belegen, dass E434 einen so starken geschmacksmodulierenden Effekt aufwiesen, dass der bittere Nachgeschmack von SAC in Wasser sehr stark reduziert wurde.

## Patentansprüche

1. Verwendung mindestens eines lebensmitteltauglichen Polyoxyethylensorbitanfettsäureesters als Geschmacksmodulator in stofflichen Zusammensetzungen, zur Reduzierung des bitteren Geschmacks und/oder Nachgeschmacks der stofflichen Zusammensetzungen, die mindestens einen High Intensity Sweetener HIS als Süßungsmittel enthalten, **dadurch gekennzeichnet, dass** es sich bei dem HIS um Saccharin handelt.

2. Verwendung nach Anspruch 1, **dadurch gekennzeichnet, dass** es sich bei dem Ester um Mono- und/oder Tri-Fettsäurester handelt.

3. Verwendung nach einem der vorhergehenden Ansprüche, **dadurch gekennzeichnet, dass** es sich bei dem Ester um mindestens einen Mono-Fettsäureester handelt.

4. Verwendung nach einem der vorhergehenden Ansprüche, **dadurch gekennzeichnet, dass** es sich bei der Fettsäure um eine gesättigte oder ungesättigte Fettsäure handelt.

5. Verwendung nach einem der vorhergehenden Ansprüche, **dadurch gekennzeichnet, dass** es sich bei den Fettsäuren um C11 bis C18 Fettsäuren handelt.

6. Verwendung nach einem der vorhergehenden Ansprüche, **dadurch gekennzeichnet, dass** die mindestens eine Fettsäure ausgewählt aus ist der Gruppe bestehend aus Laurinsäure, Ölsäure, Palmitinsäure und Stearinsäure.

7. Verwendung nach einem der vorhergehenden Ansprüche, **dadurch gekennzeichnet, dass** es sich bei der Fettsäure um Palmitinsäure handelt

8. Verwendung nach einem der vorhergehenden Ansprüche, **dadurch gekennzeichnet, dass** ein Polyoxyethylensorbitanmonopalmitinsäurester verwendet wird.

9. Verwendung nach einem der vorhergehenden Ansprüche, **dadurch gekennzeichnet, dass** 0,0005 bis 0,005 Gew.-%, insbesondere 0,001 Gew.-%, Fettsäureester verwendet werden.

10. Verwendung nach einem der vorhergehenden Ansprüche, **dadurch gekennzeichnet, dass** es sich bei den stofflichen Zusammensetzungen um Lebensmittel, Getränke, Genussmittel, Süßungsmittel, Tiernahrungsmittel, Kosmetika und Pharmazeutika handelt.

11. Verwendung nach einem der vorhergehenden Ansprüche, **dadurch gekennzeichnet, dass** es sich bei der stofflichen Zusammensetzung um eine zuckerarme Zusammensetzung mit weniger als 10 g Zucker pro Liter bzw. kg Zusammensetzung, insbesondere um eine zuckerfreie Zusammensetzung, handelt.

12. Verwendung nach einem der vorhergehenden Ansprüche, **dadurch gekennzeichnet, dass** es sich um eine kohlenhydratarme, insbesondere kohlenhydratfreie, Zusammensetzung handelt.

13. Verwendung nach einem der vorhergehenden Ansprüche, **dadurch gekennzeichnet, dass** es sich um eine fettfreie Zusammensetzung handelt.

14. Verwendung nach einem der vorhergehenden Ansprüche, **dadurch gekennzeichnet, dass** es sich bei der stofflichen Zusammensetzung um ein, insbesondere koffeinhaltiges, Saccharin gesüßtes und zuckerfreies Getränk handelt.

15. Verwendung nach einem der vorhergehenden Ansprüche, **dadurch gekennzeichnet, dass** die Polyoxyethylensorbitanfettsäureester der Reduzierung des bitteren Geschmacks und/oder Nachgeschmack eines Saccharis gesüssten und zuckerfreien Getränkes dienen.

16. Verfahren zur Reduzierung des bitteren Geschmacks und/oder Nachgeschmacks von stofflichen Zusammensetzungen, die mindestens einen High Intensity Sweetener HIS als Süßungsmittel enthalten, wobei es sich bei dem HIS um Saccharin handelt, **dadurch gekennzeichnet, dass** man den stofflichen Zusammensetzungen mindestens einen lebensmitteltauglichen Polyoxyethylensorbitanfettsäureester zusetzt.

17. Verfahren nach Anspruch 16, **dadurch gekennzeichnet, dass** es sich bei dem Ester um Mono- oder Tri-Fettsäurester handelt.

18. Verfahren nach einem der Ansprüche 16-17, **dadurch gekennzeichnet, dass** es sich bei dem Ester um einen Monofettsäureester handelt.

19. Verfahren nach einem der Ansprüche 16-18, **dadurch gekennzeichnet, dass** es sich bei den Fettsäuren um C11 bis C18 Fettsäuren handelt.

20. Verfahren nach einem der Ansprüche 16-19, **dadurch gekennzeichnet, dass** es sich bei der Fettsäure um eine gesättigte oder ungesättigte Fettsäure handelt.

21. 22. Verfahren nach einem der Ansprüche 16-20, **dadurch gekennzeichnet, dass** die Fettsäure ausgewählt aus ist der Gruppe bestehend aus Laurinsäure, Ölsäure, Palmitinsäure und Stearinsäure.

22. Verfahren nach einem der Ansprüche 16-24, **dadurch gekennzeichnet, dass** es sich bei der Fettsäure um Palmitinsäure handelt

23. Verfahren nach einem der Ansprüche 16-17, **dadurch gekennzeichnet, dass** ein Polyoxyethylensorbitanmonopalmitinsäurester verwendet wird.

24. Verfahren nach einem der Ansprüche 16-23, **dadurch gekennzeichnet, dass** der mindestens eine Polyoxyethylensorbitanfettsäureester in einer Menge zugesetzt wird, dass in den stofflichen Zusammensetzungen eine Konzentration von 1 bis 100 mg, insbesondere 10 bis 15 mg, vorzugsweise 13 mg, pro Liter bzw. kg Zusammensetzung resultiert.

25. Verfahren nach einem der Ansprüche 16-24, **dadurch gekennzeichnet, dass** die stofflichen Zusammensetzungen Lebensmittel, Getränke, Genussmittel, Süßungsmittel, Tiernahrungsmittel, Kosmetika und Pharmazeutika sind.

26. Verfahren nach einem der Ansprüche 16-25, **dadurch gekennzeichnet, dass** es sich bei den stofflichen Zusammensetzungen um zuckerarme Zusammensetzungen mit weniger als 10 g Zucker pro Liter bzw. kg Zusammensetzung, insbesondere um zuckerfreie Zusammensetzungen, handelt.

27. Verfahren nach einem der Ansprüche 16-26, **dadurch gekennzeichnet, dass** es sich um eine kohlenhydratarme, insbesondere kohlenhydratfreie, Zusammensetzung handelt.

28. Verfahren nach einem der Ansprüche 16-27, **dadurch gekennzeichnet, dass** es sich um eine fettfreie Zusammensetzung handelt.

29. Verfahren nach einem der Ansprüche 16-28, **dadurch gekennzeichnet, dass** es sich bei der stofflichen Zusammensetzung um ein, insbesondere koffeinhaltiges, saccharin gesüβtes zuckerfreies Getränk handelt.

30. Verfahren nach einem der Ansprüche 16-29, **dadurch gekennzeichnet, dass** bei der Geschmacksmodulation der bittere Geschmack und/oder Nachgeschmack eines saccharin gesüssten zuckerfreien Getränkes reduziert wird.

## Claims

1. Use of at least one polyoxyethylene sorbitan fatty acid ester which is suitable for use in foodstuff as taste modulator in compositions of matter for reducing the bitter taste and/or aftertaste of the compositions of matter which comprise at least one high intensity sweetener HIS as sweetener, **characterized in that** the HIS is saccharin.

2. The use according to claim 1, **characterized in that** the ester is a mono- and/or trifatty acid ester.

3. The use according to any one of the preceding claims, **characterized in that** the ester is at least one monofatty acid ester.

4. The use according to any one of the preceding claims, **characterized in that** the fatty acid is a saturated or unsaturated fatty acid.

5. The use according to any one of the preceding claims, **characterized in that** the fatty acids are C11 to C18 fatty acids.

6. The use according to any one of the preceding claims, **characterized in that** the at least one fatty acid is selected from the group consisting of lauric acid, oleic acid, palmitic acid and stearic acid.

7. The use according to any one of the preceding claims, **characterized in that** the fatty acid is palmitic acid.

8. The use according to any one of the preceding claims, **characterized in that** a polyoxyethylene sorbitan monopalmitic acid ester is used.

9. The use according to any one of the preceding claims, **characterized in that** 0.0005 to 0.005 % by weight, in particular 0.001 % by weight of fatty acid ester are used.

10. The use according to any one of the preceding claims, **characterized in that** the compositions of matter are foods, drinks, articles consumed for pleasure, sweeteners, animal feeds, cosmetics and pharmaceuticals.

11. The use according to any one of the preceding claims, **characterized in that** the composition of matter is a low-sugar composition having less than 10 g of sugar per liter or kg of composition, in particular a sugar-free composition.

12. The use according to any one of the preceding claims, **characterized in that** the composition is a low-carbohydrate, in particular a carbohydrate-free, composition.

13. The use according to any one of the preceding claims, **characterized in that** the composition is a fat-free composition.

14. The use according to any one of the preceding claims, **characterized in that** the composition of matter is a saccharin-sweetened and sugar-free drink, which in particular comprises caffeine.

15. The use according to any one of the preceding claims, **characterized in that** the polyoxyethylene sorbitan fatty acid esters serve the purpose of reducing the bitter taste and/or the aftertaste of a saccharin-sweetened and sugar-free drink.

16. A process for reducing the bitter taste and/or aftertaste of compositions of matter containing at least one high intensity sweetener HIS as sweetener, wherein the HIS is saccharin, **characterized in that** at least one polyoxyethylene sorbitan fatty acid ester that is suitable for use in foodstuff is added to the compositions of matter.

17. The process according to claim 16, **characterized in that** the ester is a mono- or trifatty acid ester.

18. The process according to any one of claims 16 to 17, **characterized in that** the ester is a monofatty acid ester.

19. The process according to any one of claims 16 to 18, **characterized in that** the fatty acids are C11 to C18 fatty acids.

20. The process according to any one of claims 16 to 19, **characterized in that** the fatty acid is a saturated or unsaturated fatty acid.

21. The process according to any one of claims 16 to 20, **characterized in that** the fatty acid is selected from the group consisting of lauric acid, oleic acid, palmitic acid and stearic acid.

22. The process according to any one of claims 16 to 21, **characterized in that** the fatty acid is palmitic acid.

23. The process according to any one of claims 16 to 22, **characterized in that** a polyoxyethylene sorbitan monopalmitic acid ester is used.

24. The process according to any one of claims 16 to 23, **characterized in that** the at least one polyoxyethylene sorbitan fatty acid ester is added in an amount such that a concentration of 1 to 100 mg, in particular of 10 to 15 mg, preferably of 13 mg, per liter or kg of composition results in the compositions of matter.

25. The process according to any one of claims 16 to 24, **characterized in that** the compositions of matter are foods, drinks, articles consumed for pleasure, sweeteners, animal feeds, cosmetics and pharmaceuticals.

26. The process according to any one of claims 16 to 25, **characterized in that** the compositions of matter are low-sugar compositions having less than 10 mg of sugar per liter or kg of composition, in particular sugar-free compositions.

27. The process according to any one of claims 16 to 26, **characterized in that** the composition is a low-carbohydrate composition, in particular a carbohydrate-free composition.

28. The process according to any one of claims 16 to 27, **characterized in that** the composition is a fat-free composition.

29. The process according to any one of claims 16 to 28, **characterized in that** the composition of matter is a saccharin-sweetened and sugar-free drink, which in particular comprises caffeine.

30. The process according to any one of claims 16 to 29, **characterized in that** through the taste modulation the bitter taste and/or aftertaste of a saccharin-sweetened sugar-free drink is reduced.

## Revendications

1. Utilisation d'au moins un ester d'acide gras de polyoxyéthylène sorbitane de qualité alimentaire à titre de modulateur d'arôme dans des compositions de substances, dans le but de réduire le goût et/ou l'arrière-goût amer des compositions de substances, qui comprennent au moins un édulcorant de haute intensité (HIS) à titre d'agent sucrant, **caractérisée en ce que** le HIS est la saccharine.

2. Utilisation selon la revendication 1, **caractérisée en ce que** l'ester est un mono- et/ou triester d'acide gras.

3. Utilisation selon l'une des revendications précédentes, **caractérisée en ce que** l'ester est au moins un monoester d'acide gras.

4. Utilisation selon l'une des revendications précédentes, **caractérisée en ce que** l'acide gras est un acide gras saturé ou insaturé.

5. Utilisation selon l'une des revendications précédentes, **caractérisée en ce que** les acides gras sont des acides gras en C11 à C18.

6. Utilisation selon l'une des revendications précédentes, **caractérisée en ce que** le au moins un acide gras est choisi dans le groupe constitué par l'acide laurique, l'acide oléique, l'acide palmitique et l'acide stéarique.

7. Utilisation selon l'une des revendications précédentes, **caractérisée en ce que** l'acide gras est l'acide palmitique.

8. Utilisation selon l'une des revendications précédentes, **caractérisée en ce que** l'on utilise un monoester d'acide palmitique de polyoxyéthylène sorbitane.

9. Utilisation selon l'une des revendications précédentes, **caractérisée en ce que** l'on utilise 0,0005 à 0,005 % en poids, en particulier 0,001 % en poids d'ester d'acide gras.

10. Utilisation selon l'une des revendications précédentes, **caractérisée en ce que** les compositions de substances sont des produits alimentaires, des boissons, des produits de luxe, des agents sucrants, des produits destinés à l'alimentation animale, des cosmétiques et des produits pharmaceutiques.

11. Utilisation selon l'une des revendications précédentes, **caractérisée en ce que** la composition de substances est une composition pauvre en sucres avec moins de 10 g de sucre par litre ou kg de composition, en particulier une composition sans sucres.

12. Utilisation selon l'une des revendications précédentes, **caractérisée en ce que** c'est une composition pauvre en hydrates de carbone, en particulier sans hydrates de carbone.

13. Utilisation selon l'une des revendications précédentes, **caractérisée en ce que** c'est une composition sans graisses.

14. Utilisation selon l'une des revendications précédentes, **caractérisée en ce que** la composition de substances est une boisson sans sucre, édulcorée à la saccharine, contenant en particulier de la caféine.

15. Utilisation selon l'une des revendications précédentes, **caractérisée en ce que** les esters d'acide gras de polyoxyéthylène sorbitane servent à réduire le goût et/ou l'arrière-goût amer d'une boisson sans sucres édulcorée à la saccharine.

16. Procédé de réduction du goût et/ou de l'arrière-goût amer de compositions de substances qui comprennent au moins un édulcorant de haute intensité HIS à titre d'agent sucrant, le HIS étant la saccharine, **caractérisé en ce que** l'on ajoute aux compositions de substances au moins un ester d'acide gras de polyoxyéthylène sorbitane de qualité alimentaire.

17. Procédé selon la revendication 16, **caractérisé en ce que** l'ester est un mono- et/ou triester d'acide gras.

18. Procédé selon l'une des revendications 16 à 17, **caractérisé en ce que** l'ester est un monoester d'acide gras.

19. Procédé selon l'une des revendications 16 à 18, **caractérisé en ce que** les acides gras sont des acides gras en C11 à C18.

20. Procédé selon l'une des revendications 16 à 19, **caractérisé en ce que** l'acide gras est un acide gras saturé ou insaturé.

21. Procédé selon l'une des revendications 16 à 20, **caractérisé en ce que** l'acide gras est choisi dans le groupe constitué par l'acide laurique, l'acide oléique, l'acide palmitique et l'acide stéarique.

22. Procédé selon l'une des revendications 16 à 21, **caractérisé en ce que** l'acide gras est l'acide palmitique.

23. Procédé selon l'une des revendications 16 à 22, **caractérisé en ce que** l'on utilise un monoester d'acide palmitique de polyoxyéthylène sorbitane.

24. Procédé selon l'une des revendications 16 à 23, **caractérisé en ce que** le au moins un ester d'acide gras de polyoxyéthylène sorbitane est ajouté en une quantité telle qu'il en résulte une concentration de 1 à 100 mg, en particulier de 10 à 15 mg, de préférence de 13 mg, par litre ou kg de composition dans les compositions de substances.

25. Procédé selon l'une des revendications 16 à 24, **caractérisé en ce que** les compositions de substances sont des produits alimentaires, des boissons, des produits de luxe, des agents sucrants, des produits destinés à l'alimentation animale, des cosmétiques et des produits pharmaceutiques.

26. Procédé selon l'une des revendications 16 à 25, **caractérisé en ce que** les compositions de substances sont des compositions pauvres en sucres avec moins de 10 g de sucre par litre ou kg de composition, en particulier des compositions sans sucres.

27. Procédé selon l'une des revendications 16 à 26, **caractérisé en ce que** c'est une composition pauvre en hydrates de carbone, en particulier sans hydrates de carbone.

28. Procédé selon l'une des revendications 16 à 27, **caractérisé en ce que** c'est une composition sans graisses.

29. Procédé selon l'une des revendications 16 à 28, **caractérisé en ce que** la composition de substances est une boisson sans sucres, édulcorée à la saccharine, contenant en particulier de la caféine.

30. Procédé selon l'une des revendications 16 à 29, **caractérisé en ce que** la modulation du goût permet de réduire le goût et/ou l'arrière-goût amer d'une boisson sans sucres édulcorée à la saccharine.
